# EUROPEAN PATENT SPECIFICATION

(11) **EP 3 508 246 B1**
(45) Date of publication and mention of the grant of the patent: **11.08.2021**
(21) Application number: 16834166.7
(22) Date of filing: 05.09.2016
(51) Int. Cl.: A61M 25/09

(54) **GUIDE WIRE**
FÜHRUNGSDRAHT
FIL-GUIDE

(43) Date of publication of application: 10.07.2019
(73) Proprietor: ASAHI INTECC CO., LTD., Seto-shi, Aichi 489-0071 (JP)
(72) Inventor: USHIDA, Keisuke, Seto-shi, Aichi 489-0071 (JP); YOSHIDA, Toshiyuki, Seto-shi, Aichi 489-0071 (JP)
(74) Representative: Winter, Brandl - Partnerschaft mbB
(86) International application number: PCT/JP2016/075978
(87) International publication number: WO 2018/042652

(56) References cited:
- WO-A1-92/07507
- JP-A- H0 351 060
- US-A- 5 549 580
- US-A- 5 549 580
- US-A- 5 830 155
- US-A1- 2004 064 069
- US-A1- 2004 082 879
- US-A1- 2009 299 332

## Description

### TECHNICAL FIELD

The present invention relates to a guidewire to be inserted into a blood vessel and a digestive organ.

### BACKGROUND ART

A narrowed or obstructed segment formed in a blood vessel, bile duct, pancreatic duct, and the like usually results in a restricted flow of blood, bile (gall), and pancreatic fluid. Therapeutic procedures using a catheter are widely performed as a measure for treating such a narrowed or obstructed segment.

A guidewire is commonly used in order to guide a catheter to a narrowed or obstructed segment. An example of a guidewire can be found in document US-A-5549580. A high frictional resistance between a guidewire and a vessel wall of a blood vessel, bile duct, pancreatic duct and the like may result in difficult insertion of the guidewire into a narrowed or obstructed segment by an operator. Accordingly, known is a guidewire including a resin layer covering the outer periphery of a coil body throughout the entire length of the coil body for enabling easy insertion of the guidewire into a narrowed or obstructed segment (see Patent Documents 1 and 2 below).

In the guidewire according to Patent Document 1, the outer periphery of a coil body is covered with a resin film (resin layer) throughout its entire length. Similarly, in the guidewire according to Patent Document 2, the outer periphery of a coil body is covered with a resin coating layer throughout its entire length.

These guidewires can reduce frictional resistance, and thus allows easier insertion of the guidewires into narrowed or obstructed segments. However, they have the following disadvantages: the rotation performance of a guidewire is poor (the operativity of a guidewire is poor) ; and the stiffness of the distal end part of a guidewire tends to be high.

### CITATION LIST

### PATENT DOCUMENT

Patent Document 1: Japanese Patent Application Laid-Open No. 2007-75531
Patent Document 2 : Japanese Patent Application Laid-Open No. 2008-307367

### SUMMARY OF THE INVENTION

### TECHNICAL PROBLEM

The present invention is made in view of these circumstances . An obj ect of the present invention is to provide a guidewire having improved rotation performance of the guidewire (improved operativity of the guidewire) while maintaining the insertability of the guide, and having a reduced risk of detachment (peeling off) from the distal end of a resin layer.

### SOLUTION TO PROBLEM

The above object can be achieved by virtue of the means recited below.

A first aspect of the present invention provides a guidewire as defined in claim 1.

A second aspect of the present invention provides the guidewire according to the first aspect, in which the distal end part of the resin layer is fixed to the core shaft.

A third aspect of the present invention provides the guidewire according to the first or second aspect, in which the distal end part of the resin layer is provided with a bulged portion having an outer diameter larger than that of the distal end part.

The fourth aspect of the present invention provides the guidewire according to any one of the first to third aspects, including a fixation portion fixing the core shaft, the coil body, and the resin layer at a boundary between the tapered part of the resin layer and the proximal end part of the resin layer.

The fifth aspect of the present invention provides the guidewire according to any one of the first to fourth aspects, in which the coil body is formed by spirally winding multiple wires, and the distal end part of the resin layer enters into a space between adjacent wires of the multiple wires of the coil body as viewed in a cross sectional view.

A sixth aspect of the present invention provides the guidewire according to any one of the first to fifth aspects, in which the coil body includes a small-diameter portion having a small coil outer diameter and being located at the proximal end part of the resin layer, a large-diameter portion having a coil outer diameter larger than that of the small-diameter portion and being located at the distal end part of the resin layer, and an increasing-diameter portion having a coil outer diameter increasing toward the distal side and being located at the tapered part of the resin layer, and the coil outer diameter at the large-diameter portion of the coil body is larger than the large outer diameter at the proximal end part of the resin layer.

### EFFECT OF THE INVENTION

In the first aspect of the present invention, a resin layer is included a proximal end part having a large outer diameter, the proximal end part covering an outer periphery of the coil body throughout the entire length of the proximal end part, a distal end part having an outer diameter smaller than that of the proximal end part in a distal side relative to the proximal end part, the distal end part being located between the core shaft and the coil body, and a tapered part having an outer diameter decreasing toward the distal side between the proximal end part and the distal end part, in which the coil body is exposed without being covered with the resin layer at the distal end part. This can improve the rotation performance of the guidewire (the operativity of the guidewire) while maintaining the insertability of the guidewire, and can reduce a risk of detachment (peeling off) of the resin layer from the distal end.

In the second aspect of the present invention, the distal end part of the resin layer is fixed to the core shaft, and thus a risk of outward projection of the distal end of the resin layer and a risk of detachment (peeling off) of the resin layer from the distal end can be reduced even when the guidewire is inserted into a curved blood vessel, bile duct, pancreatic duct, and the like.

In the guidewire according to the third aspect of the present invention, the bulged portion having an outer diameter larger than that of the distal end part is provided at the distal end part of the resin layer. Therefore, the bulged portion provided at the distal end part of the resin layer can be caught at the inner periphery surface of the coil body even in a case where the resin layer is being detached (peeled off) from the distal end upon insertion of the guidewire into a curved blood vessel, bile duct, pancreatic duct, and the like. This can further reduce a risk of detachment (peeling off) of the resin layer by virtue of the anchoring effect.

In the guidewire according to the fourth aspect of the present invention, the fixation portion fixing the core shaft, the coil body, and the resin layer is provided at a boundary of the tapered part of the resin layer and the proximal end part of the resin layer. When a guidewire is inserted to a narrowed or obstructed segment, frictional resistance with the vessel wall of a blood vessel, bile duct, pancreatic duct, and the like becomes high at the boundary of the tapered part of the resin layer and the proximal end part of the resin layer. Detachment (peeling off) of the resin layer at the boundary of the tapered part and proximal end part upon insertion of the guidewire can be reduced because the resin layer is preliminarily fixed to the coil body and the core shaft through the fixation portion.

In the guidewire according to the fifth aspect of the present invention, the coil body is formed by spirally winding multiple wires, and the distal end part of the resin layer enters into a space between adjacent wires of the multiple wires of the coil body as view in an cross sectional view. Therefore, detachment (peeling off) of the resin layer can be further reduced by virtue of the anchor effect between the distal end part of the resin layer and the coil body.

In the guidewire according to the sixth aspect of the present invention, the coil body includes a small-diameter portion having a small coil outer diameter and being located at the proximal end part of the resin layer; a large-diameter portion having a coil outer diameter larger than that of the small-diameter portion and being located at the distal end part of the resin layer; and an increasing-diameter portion having a coil outer diameter increasing toward the distal side and being located at the tapered part of the resin layer, in which the coil outer diameter at the large-diameter portion of the coil body is larger than that at the proximal end part of the resin layer. This can reduce a risk of contacting the boundary of the tapered part of the resin layer and the proximal end part of the resin layer with the vessel wall of a blood vessel, bile duct, pancreatic duct, and the like even when a guidewire is inserted into a curved blood vessel, bile duct, pancreatic duct, and the like. As a result, detachment (peeling off) of the resin layer at the boundary of the tapered part and proximal end part can be further reduced upon insertion of the guidewire.

### BRIEF DESCRIPTION OF THE DRAWINGS

Fig. 1 shows a guidewire according to the first embodiment.
Fig. 2 shows a cross sectional view at A-A of Fig. 1.
Fig. 3 shows a cross sectional view at B-B of Fig. 1.
Fig. 4 shows a cross sectional view at C-C of Fig. 1.
Fig. 5 shows a guidewire according to the second embodiment.
Fig. 6 shows a guidewire according to the third embodiment.
Fig. 7 shows a guidewire according to the fourth embodiment, which is a variation of Fig. 6.
Fig. 8 shows a guidewire according to the fifth embodiment.
Fig. 9 shows a guidewire according to the sixth embodiment.
Fig. 10 shows a cross sectional view at D-D of Fig. 9.
Fig. 11 shows a Fig. 10-equivalent cross sectional view of a guidewire according to the seventh embodiment.
Fig. 12 shows a guidewire according to the eighth embodiment.

### DESCRIPTION OF EMBODIMENTS

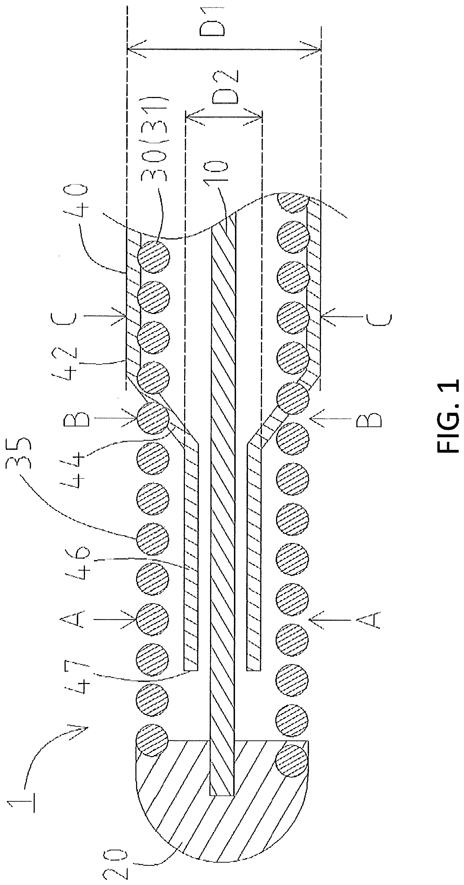

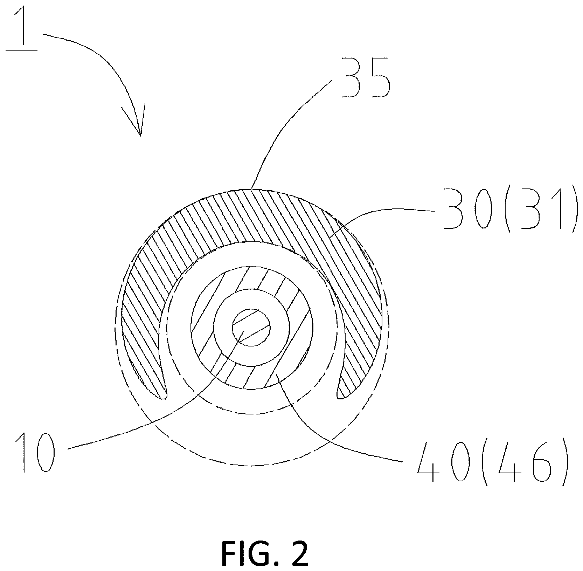

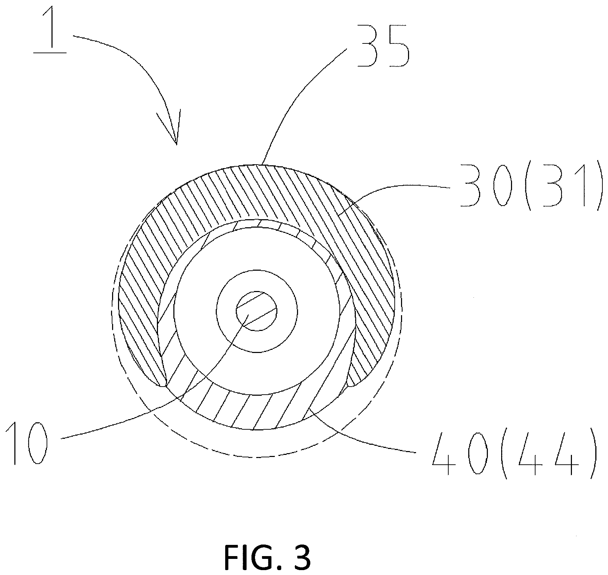

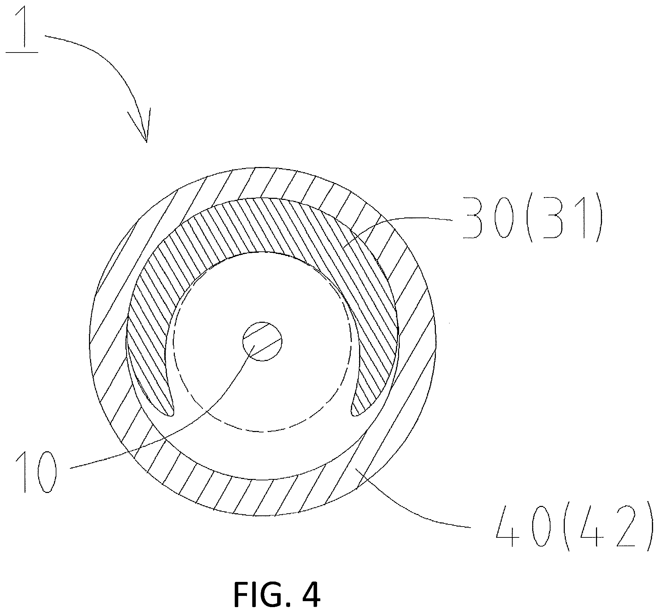

A guidewire 1 according to the first embodiment will be described with reference to Figs. 1 to 4. The left side in Fig. 1 corresponds to the distal side (the front side) which is to be inserted into the body, and the right side corresponds to the proximal side (the tail side) which is to be operated by an operator such as a physician. Fig. 2 shows the A-A cross section of Fig. 1, and Fig. 3 shows the B-B cross section of Fig. 1, and Fig. 4 shows the C-C cross section of Fig. 1.

The guidewire 1 is, for example, intended for use in guiding a catheter to a narrowed or obstructed segment. As shown in Fig. 1, the guidewire 1 mainly includes a core shaft 10, a distal end fixed part 20, a coil body 30, and a resin layer 40.

The core shaft 10 is made of stainless steel (SUS 304, SUS 316, and the like) , a superelastic alloy of a Ni-Ti alloy, or the like.

The coil body 30 is formed by winding a single wire 31 around the outer periphery of the core shaft 10, the single wire 31 being radiopaque. Materials which can be used for the wire 31 of the coil body 30 include, for example, gold, platinum, tungsten, and alloys of these elements. In a case where the coil body 30 is formed with the wire 31 having radiopacity, an operator can detect the position of the coil body 30 under radiography imaging.

The distal end fixed part 20 fixes the distal end of the core shaft 10 to the distal end of the coil body 30, and is formed with a solder material (an aluminum alloy solder, a silver solder, a gold solder, and the like) or a metal solder (an Au-Sn alloy, an Ag-Sn alloy, and the like).

The resin layer 40 includes a proximal end part 42 having a large outer diameter D1 and covering the outer periphery of the coil body 30; a distal end part 46 having an outer diameter smaller than that of the proximal end part 42 and being located between the core shaft 10 and the coil body 30 in the distal side relative to the proximal end part 42; and a tapered part 44 having an outer diameter decreasing from D1 to D2 toward the distal side between the proximal end part 42 and the distal end part 46.

Materials which can be used for the resin layer 40 as described above include polyurethane, polyethylene, polyvinyl chloride, polyester, polypropylene, polyamide, fluorinated resin such as PTFE, silicon resin, and the like.

As shown in Figs. 1 and 2, the distal end part 46 of the resin layer 40 is located between the core shaft 10 and the coil body 30. As shown in Figs. 1 and 3, the tapered part 44 of the resin layer 40 is located inside the coil body 30 in the distal side, but located at the outer periphery of the coil body 30 in the tail side. As shown in Figs. 1 and 4, the proximal end part 42 of the resin layer 40 is located at the outer periphery of the coil body 30.

An outer periphery surface 35 of the coil body 30 is exposed without being covered with the resin layer 40 in the distal side, but covered with the tapered part 44 and the proximal end part 42 of the resin layer 40 in the proximal side (see Fig. 1). Accordingly, the coil body 30 at the distal end part 46 of the resin layer 40 is exposed without being covered with the resin layer 40.

This can improve the rotation performance of the guidewire 1 (the operativity of the guidewire 1) by virtue of the coil body 30 which is exposed without being covered with the resin layer 40 while maintaining the insertability of the guidewire 1 by virtue of the proximal end part 42 of the resin layer 40. Further, a risk of detachment (peeling off) of the resin layer 40 from the distal end 47 can be reduced upon insertion of the guidewire 1 into a blood vessel, bile duct, pancreatic duct, and the like because the distal end part 46 of the resin layer 40 is located inside the coil body 30.

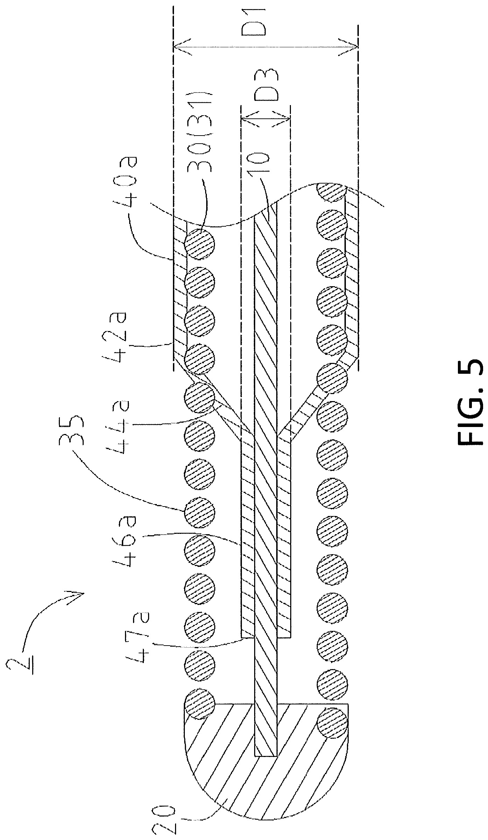

Next, a guidewire 2 according to the second embodiment will be described with reference to Fig. 5. Only differences from the guidewire 1 as shown in Fig. 1 will be described. In the guidewire 2, a resin layer 40a includes a proximal end part 42a having the large outer diameter D1 and covering the outer periphery of the coil body 30; a distal end part 46a having an outer diameter D3 smaller than that of the proximal end part 42a and being located between the core shaft 10 and the coil body 30 in the distal side relative to the proximal end part 42a; and a tapered part 44a having an outer diameter decreasing from D1 to D3 toward the distal side between the proximal end part 42a and the distal end part 46a. Further, the distal end part 46a of the resin layer 40a is fixed to the core shaft 10.

This can reduce a risk of outward projection of a distal end 47a of the resin layer 40a from between the wires 31 of the coil body 30, and a risk of detachment (peeling off) of the resin layer 40a from the distal end 47a even when the guidewire 2 is inserted into a curved blood vessel, bile duct, pancreatic duct, and the like.

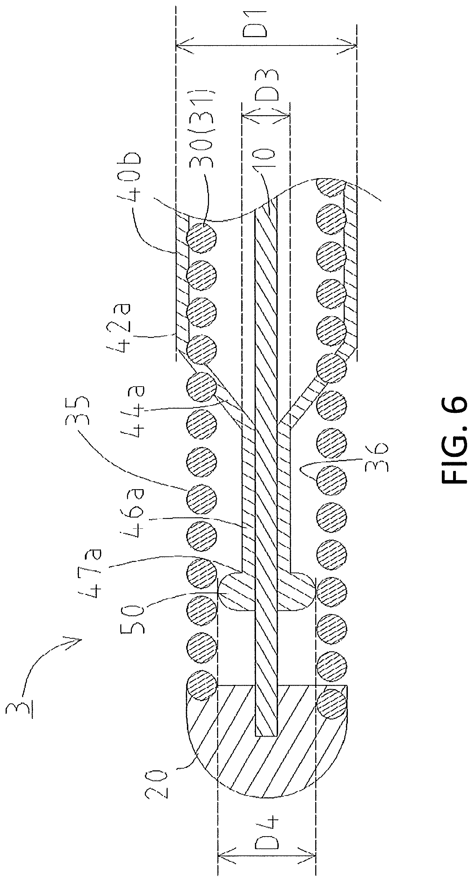

Next, a guidewire 3 according to the third embodiment will be described with reference to Fig. 6. Only differences from the guidewire 2 as shown in Fig. 5 will be described. In the guidewire 3, a resin layer 40b includes the proximal end part 42a, the tapered part 44a, and the distal end part 46a, in which a bulged portion 50 having an outer diameter D4 larger than the outer diameter D3 of the distal end part 46a is provided at the distal end 47a of the distal end part 46a. Further, the distal end part 46a of the resin layer 40b and the bulged portion 50 are fixed to the core shaft 10.

In the guidewire 3, the bulged portion 50 is integrally formed of the same material as the resin layer 40b, but the configuration is not limited to this. The bulged portion 50 may be formed of a material which is different from that of the distal end part 46a of the resin layer 40b.

The bulged portion 50 is provided at the distal end part 47a of the resin layer 40b. Thereby, the bulged portion 50 provided at the distal end part 46a of the resin layer 40b can be caught at an inner periphery surface 36 of the coil body 30 even when the resin layer 40b is being detached (peeled off) from the distal end 47a upon insertion of the guidewire 3 into a curved blood vessel, bile duct, pancreatic duct, and the like. This can further reduce a risk of detachment (peeling off) of the resin layer 40b by virtue of the anchoring effect.

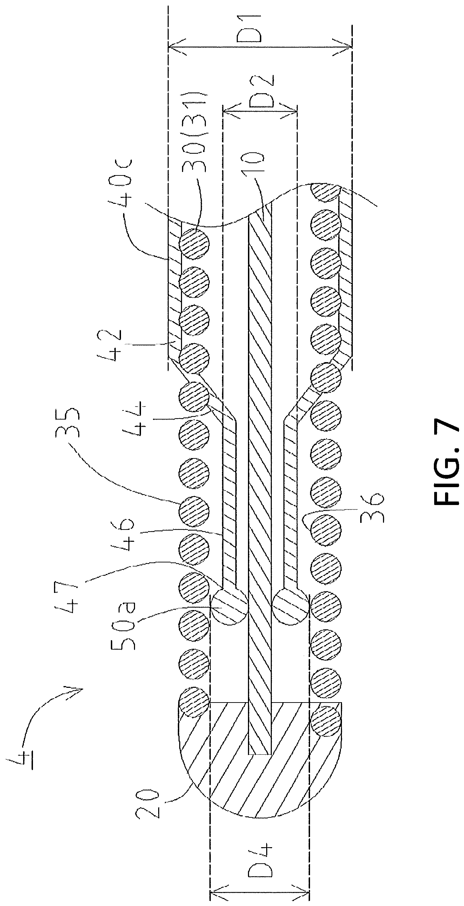

Note that in the guidewire 3 as shown in Fig. 6, the distal end part 46a of the resin layer 40b and the bulged portion 50 are fixed to the core shaft 10. The configuration, however, is not limited to this. Only a bulged portion 50a may be fixed to the core shaft 10 as shown in a guidewire 4 according to the fourth embodiment in Fig. 7. Only differences from the guidewire 1 as shown in Fig. 1 will be described. In the guidewire 4, a resin layer 40c includes the proximal end part 42, the tapered part 44, and the distal end part 46, in which the bulged portion 50a having the outer diameter D4 larger than the outer diameter D2 of the distal end part 46 is provided at the distal end 47 of the distal end part 46.

In the guidewire 4, the bulged portion 50a is integrally formed of the same material as the resin layer 40c, but the configuration is not limited to this. The bulged portion 50a may be formed of a material which is different from that of the distal end part 46 of the resin layer 40c.

The bulged portion 50a is provided at the distal end part 47 of the resin layer 40c. Thereby, the bulged portion 50a provided at the distal end part 46 of the resin layer 40c can be caught at the inner periphery surface 36 of the coil body 30 even when the resin layer 40c is being detached (peeled off) from the distal end 47 upon insertion of the guidewire 4 into a curved blood vessel, bile duct, pancreatic duct, and the like. This can further reduce a risk of detachment (peeling off) of the resin layer 40c by virtue of the anchoring effect.

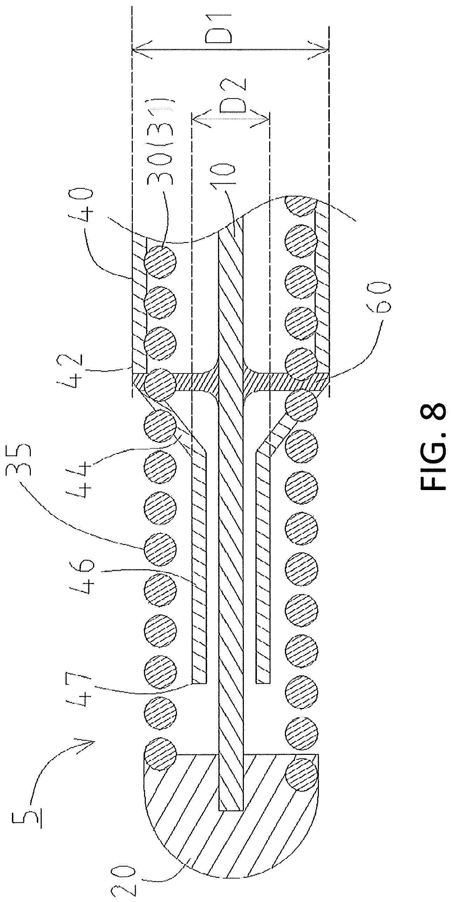

Next, a guidewire 5 according to the fifth embodiment will be described with reference to Fig. 8. Only differences from the guidewire 1 as shown in Fig. 1 will be described. In the guidewire 5, a fixation portion 60 fixing the core shaft 10 to the coil body 30 is provided at a boundary of the tapered part 44 of the resin layer 40 and the proximal end part 42 of the resin layer 40.

In the guidewire 5, the resin layer 40 is pre-fixed to the coil body 30 and the core shaft 10 through the fixation portion 60. This can further reduce a risk of detachment (peeling off) of the resin layer 40 at boundary of the tapered part 44 and the proximal end part 42 upon insertion of the guidewire 5 even when frictional resistance with the vessel wall of a blood vessel, bile duct, pancreatic duct, and the like becomes high at the boundary of the tapered part 44 of the resin layer 40 and the proximal end part 42 of the resin layer 40 upon insertion of the guidewire 5 into a narrowed or obstructed segment.

Note that an adhesive is used as the fixation portion 60 in the guidewire 5, but there is no particular limitation for the material thereof.

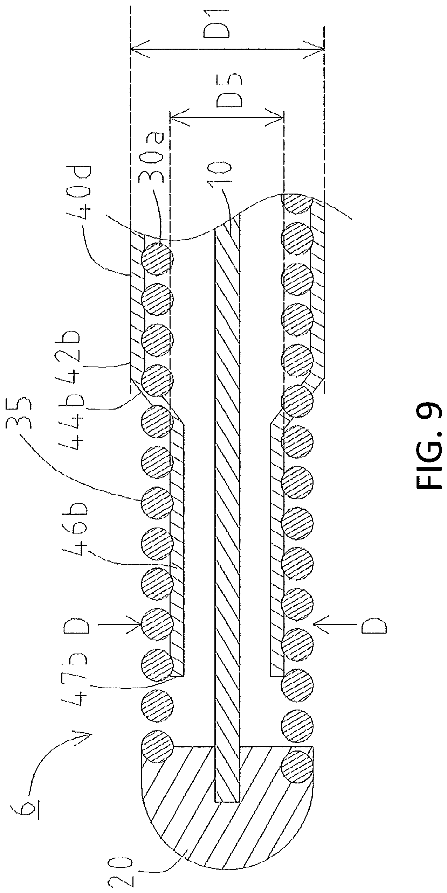

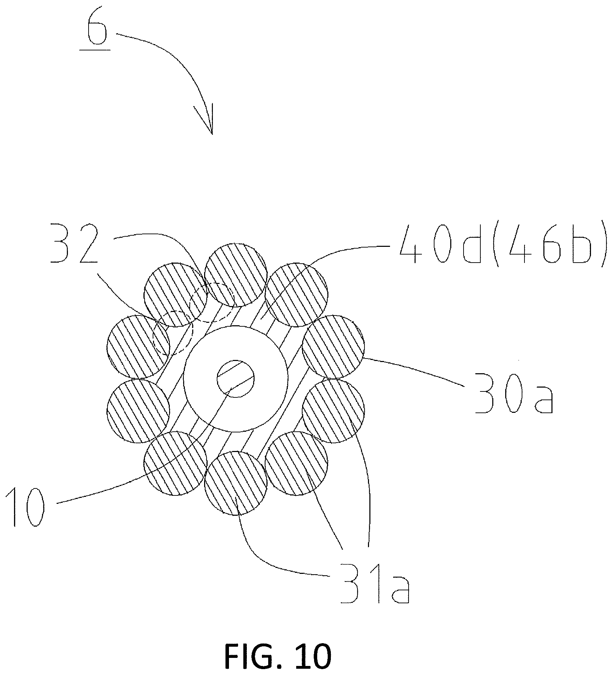

Next, a guidewire 6 according to the sixth embodiment will be described with reference to Fig. 9. Only differences from the guidewire 1 as shown in Fig. 1 will be described. In the guidewire 6, a coil body 30a is formed by spirally winding ten wires 31a (see Fig. 10). Further, as shown in Fig. 9, a resin layer 40d includes a proximal end part 42b having the large outer diameter D1 and covering the outer periphery of the coil body 30a; a distal end part 46b having an outer diameter D5 smaller than that of the proximal end part 42b and being located between the core shaft 10 and the coil body 30a in the distal side relative to the proximal end part 42b; and a tapered part 44b having an outer diameter decreasing from D1 to D5 toward the distal side between the proximal end part 42b and the distal end part 46b.

As shown in Fig. 10 which represents the D-D cross section of Fig. 9, the distal end part 46b of the resin layer 40d enters into a space 32 between adjacent wires of the multiple wires 31a of the coil body 30a as viewed in a cross sectional view.

This can further reduce a risk of detachment (peeling off) of the distal end part 46b of the resin layer 40 by virtue of the anchoring effect between the distal end part 46b of the resin 40d and the coil body 30a (in other words, because the distal end part 46b of the resin layer 40d can be caught at the space 32 between adjacent wires of the multiple wires 31a).

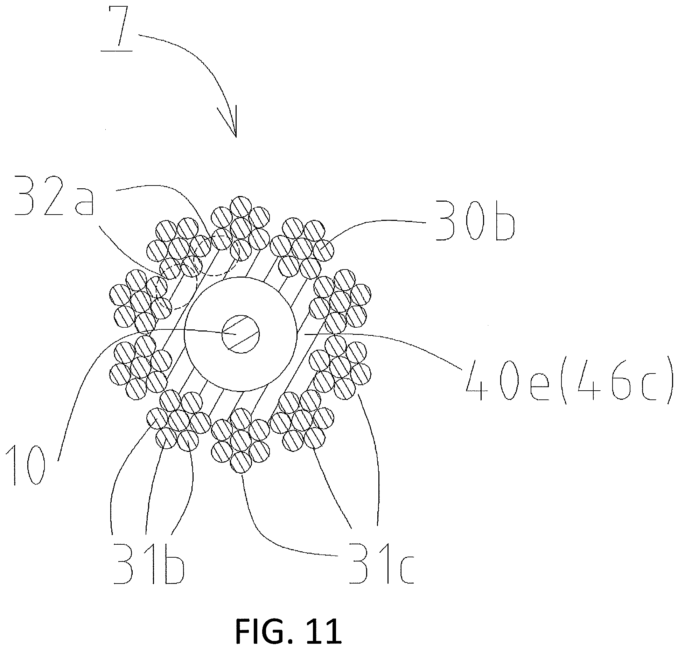

Note that in the guidewire 6 as shown in Fig. 10, the coil body 30a is formed by spirally winding ten wires 31a. However, the configuration is not limited to this. As shown in a guidewire 7 according to the seventh embodiment of Fig. 11, a coil body 30b may be used in place of the coil body 30a, the coil body 30b being formed by bundling and spirally winding ten twisted wires 31c, the twisted wires 31c being pre-prepared by spirally winding seven wires 31b. Here, as shown in Fig. 11, a distal end part 46c of a resin layer 40e enters into a space 32a between adjacent twisted wires of the twisted wires 31c of the coil body 30b.

This can further reduce a risk of detachment (peeling off) of the distal end part 46c of the resin layer 40e by virtue of the anchoring effect between the distal end part 46c of the resin 40e and the coil body 30b (in other words, because the distal end part 46c of the resin layer 40e can be caught at the space 32a between adjacent twisted wires of the twisted wires 31c) in the guidewire 7.

Materials which can be used for the multiple wires 31a of the coil body 31a or the multiple wires 31b of the coil body 31b include, for example, gold, platinum, tungsten, and alloys of these elements as in the wires 30 of the guidewire 1. Note that the number of the multiple wires 31a of the coil body 30a is not limited to ten. The number may be any as long as it is more than one. Further, the number of the twisted wires 31c of the coil body 30b is not limited to ten, either. The number may be any as long as it is more than one. Furthermore, the number of the multiple wires 31b of the twisted wires 31c as described above is not limited to seven, either. The number may be any as long as it is more than one.

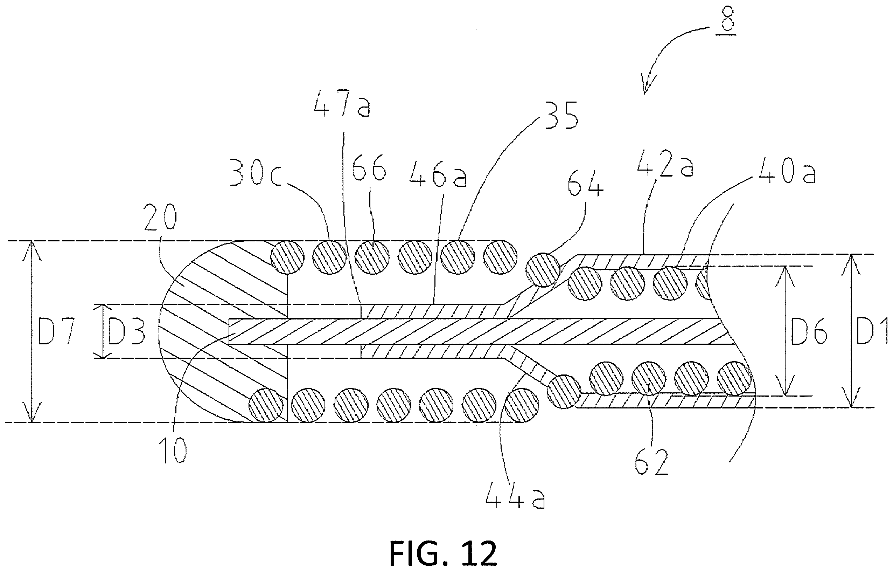

Next, a guidewire 8 according to the eighth embodiment will be described with reference to Fig. 12. Only differences from the guidewire 2 as shown in Fig. 5 will be described. In the guidewire 8, a coil body 30c includes a small-diameter portion 62 having a small coil outer diameter D6 and being located at the proximal end part 42a of the resin layer 40a; a large-diameter portion 66 having a coil outer diameter D7 larger than that of the small-diameter portion 62 and being located at the distal end part 46a of the resin layer 40a; and an increasing-diameter portion 64 having a coil outer diameter increasing from D6 to D7 toward the distal side and being located at the tapered part 44a of the resin layer 40a. Further, the coil outer diameter D7 at the large-diameter portion 66 of the coil body 30c is larger than the outer diameter D1 at the proximal end part 42a of the resin layer 40a (D7 > D1).

This can reduce a risk of contacting the boundary of the tapered part 44a of the resin layer 40a and proximal end part 42a of the resin layer 40a with the vessel wall of a blood vessel, bile duct, pancreatic duct, and the like even when the guidewire 8 is inserted into a curved blood vessel, bile duct, pancreatic duct, and the like. As a result, detachment (peeling off) of the resin layer 40a can be further reduced upon insertion of the guidewire 8.

Note that in guidewires 5, 6, 7, 8, the bulged portions 50, 50a may be provided at the distal ends 47, 47a, 47b of the distal end parts 46, 46a, 46b, 46c as in the guidewires 3, 4.

Further, in the guidewires 2, 3, 4, 6, 7, 8, the fixation portion 60 fixing the core shaft 10, the coil bodies 30, 30a, 30b, 30c, and the resin layers 40a to 40e may be provided at the boundary of the tapered parts 44a, 44b and the proximal end parts 42a, 42b as in the guidewire 5.

Furthermore, in the guidewires 1, 3, 4, 5, 6, 7, the coil bodies 30, 30a, 30b include the small-diameter portion 62 having the small coil outer diameter D6 and being located at the proximal end parts 42, 42a, 42b of the resin layers 40 to 40e; the large-diameter portion 66 having the coil outer diameter D7 larger than that of the small-diameter portion 62 and being located at the distal end parts 46 to 46c of the resin layers 40 to 40e; and the increasing-diameter portion 64 having a coil outer diameter increasing from D6 to D7 toward the distal side and being located at the tapered parts 44, 44a, 44b of the resin layers 40 to40e, in which the coil outer diameter D7 at the large-diameter portion 66 of the coil bodies 30, 30a, 30b may be larger than the outer diameter D1 at the proximal end parts 42, 42a, 42b of the resin layers 40 to 40e (D7 > D1) as in the guidewire 8.

Note that in the guidewires 1 to 8 as described above, the distal ends 47, 47a, 47b of the distal end parts 46 to 46c of the resin layers 40 to 40e are separated from the distal end fixed part 20 toward the proximal end side, but the configuration is not limited to this. For example, the distal ends 47, 47a, 47b of the distal end parts 46 to 46c of the resin layers 40 to 40e and the distal end fixed part 20 are fixed each other.

### DESCRIPTION OF SYMBOLS

- 1 to 8: Guidewire
- 10: Core shaft
- 20: Distal end fixed part
- 30, 30a, 30b, 30c: Coil body
- 31, 31a, 31b: Wires
- 31c: Twisted wires
- 32, 32a,: Space between wires
- 35: Outer periphery surface
- 40, 40a, 40b, 40c, 40d, 40e: Resin layer
- 42, 42a, 42b: Proximal end part
- 44, 44a, 44b: Tapered part
- 46, 46a, 46b, 46c: Distal end part
- 47, 47a, 47b: Distal end
- 50,: 50a, Bulged portion
- 60: Fixation portion
- 62: Small-diameter portion
- 64: Increasing-diameter portion
- 66: Large-diameter portion

## Claims

1. A guidewire (1; 2; 3; 4; 5; 6; 7; 8), comprising:
a core shaft (10);
a coil body (30; 30a; 30b; 30c) wound around an outer periphery of the core shaft (10); and
a resin layer (40; 40a; 40b; 40c; 40d; 40e) including a proximal end part (42; 42a; 42b) having a large outer diameter (D1), a distal end part (46; 46a; 46b; 46c) having an outer diameter (D2; D3) smaller than that of the proximal end part (42; 42a; 42b) in a distal side relative to the proximal end part (42; 42a; 42b), the distal end part (46; 46a; 46b; 46c) being located between the core shaft (10) and the coil body (30; 30a; 30b; 30c), and a tapered part (44; 44a; 44b) having an outer diameter decreasing toward the distal side between the proximal end part (42; 42a; 42b) and the distal end part (46; 46a; 46b; 46c),
wherein the coil body (30; 30a; 30b; 30c) is exposed without being covered with the resin layer (40; 40a; 40b; 40c; 40d; 40e) at the distal end part (46; 46a; 46b; 46c), **characterised in that**
the proximal end part (42; 42a; 42b) covers an outer periphery of the coil body (30; 30a; 30b; 30c) throughout entire length of the proximal end part (42; 42a; 42b).

2. The guidewire (2; 3) according to claim 1, wherein the distal end part (46a) of the resin layer (40a; 40b) is fixed to the core shaft (10).

3. The guidewire (3; 4; 5; 6; 7; 8) according to claim 1 or 2, wherein a bulged portion (50; 50a) having an outer diameter (D4) larger than the outer diameter (D2; D3) of the distal end part (46; 46a; 46b; 46c) is provided at the distal end part (46; 46a; 46b; 46c) of the resin layer (40b; 40c).

4. The guidewire (2; 3; 4; 5; 6; 7; 8) according to any one of claims 1 to 3, further comprising:
a fixation portion (60) fixing the core shaft (10), the coil body (30; 30a; 30b; 30c), and the resin layer (40; 40a; 40b; 40c; 40d; 40e) at a boundary of the tapered part (44; 44a; 44b) of the resin layer (40) and the proximal end part (42; 42a;42b) of the resin layer (40).

5. The guidewire (6; 7) according to any one of claims 1 to 4, wherein:
the coil body (30a; 30b) is formed by spirally winding multiple wires (31a; 31c), and
the distal end part (46b; 46c) of the resin layer (40d; 40e) enters into a space (32; 32a) between adjacent wires (31a; 31c) of the multiple wires (31a; 31c) of the coil body (30a; 30b) as viewed in a cross sectional view.

6. The guidewire (1; 2; 3; 4; 5; 6; 7; 8) according to any one of claims 1 to 5, wherein:
the coil body (30; 30a; 30b; 30c) includes a small-diameter portion (62) having a small coil outer diameter (D6) and being located at the proximal end part (42; 42a; 42b) of the resin layer (40; 40a; 40b; 40c; 40d; 40e), a large-diameter portion (66) having a coil outer diameter (D7) larger than that of the small-diameter portion (62) and being located at the distal end part (46; 46a; 46b; 46c) of the resin layer (40; 40a; 40b; 40c; 40d; 40e), and an increasing-diameter portion (64) having a coil outer diameter increasing toward the distal side and being located at the tapered part (44; 44a; 44b) of the resin layer (40; 40a; 40b; 40c; 40d; 40e), and
the coil outer diameter (D7) at the large-diameter portion (66) of the coil body (30; 30a; 30b; 30c) is larger than the large outer diameter (D1) at the proximal end part (42; 42a; 42b) of the resin layer (40; 40a; 40b; 40c; 40d; 40e).

## Patentansprüche

1. Führungsdraht (1; 2; 3; 4; 5; 6; 7; 8) mit:
einem Kernschaft (10);
einem um den Außenumfang des Kernschafts (10) gewickelten Wicklungskörper (30; 30a; 30b; 30c); und
einer Harzschicht (40; 40a; 40b; 40c; 40d; 40e), die einen proximalen Endteil (42; 42a; 42b) mit einem großen Außendurchmesser (D1), einen zwischen dem Kernschaft (10) und dem Wicklungskörper (30; 30a; 30b; 30c) befindlichen distalen Endteil (46; 46a; 46b; 46c) mit einem im Vergleich zum proximalen Endteil (42; 42a; 42b) kleineren Außendurchmesser (D2; D3) auf einer distalen Seite relativ zum proximalen Endteil (42; 42a; 42b) und einen Verjüngungsteil (44; 44a; 44b) mit einem zur distalen Seite abnehmenden Außendurchmesser zwischen dem proximalen Endteil (42; 42a; 42b) und dem distalen Endteil (46; 46a; 46b; 46c) aufweist,
wobei der Wicklungskörper (30; 30a; 30b; 30c) an dem distalen Endteil (46; 46a; 46b; 46c) ohne Ummantelung durch die Harzschicht (40; 40a; 40b; 40c; 40d; 40e) freiliegt, **dadurch gekennzeichnet, dass**
der proximale Endteil (42; 42a; 42b) den Außenumfang des Wicklungskörpers (30; 30a; 30b; 30c) über die gesamte Länge des proximalen Endteils (42; 42a; 42b) ummantelt.

2. Führungsdraht (2; 3) nach Anspruch 1, wobei der distale Endteil (46a) der Harzschicht (40a; 40b) am Kernschaft (10) befestigt ist.

3. Führungsdraht (3; 4; 5; 6; 7; 8) nach Anspruch 1 oder 2, wobei an dem distalen Endteil (46; 46a; 46b; 46c) der Harzschicht (40b; 40c) eine Ausbauchung (50; 50a) mit einem im Vergleich zum Außendurchmesser (D2; D3) des distalen Endteils (46; 46a; 46b; 46c) größeren Außendurchmesser (D4) vorgesehen ist.

4. Führungsdraht (2; 3; 4; 5; 6; 7; 8) nach einem der Ansprüche 1 bis 3, mit:
einem Befestigungsabschnitt (60), der den Kernschaft (10), den Wicklungskörper (30; 30a; 30b; 30c) und die Harzschicht (40; 40a; 40b; 40c; 40d; 40e) an der Grenze des Verjüngungsteils (44; 44a; 44b) der Harzschicht (40) und des proximalen Endteils (42; 42a;42b) der Harzschicht (40) befestigt.

5. Führungsdraht (6; 7) nach einem Anspruch 1 bis 4, wobei:
der Wicklungskörper (30a; 30b) aus mehreren spiralförmig gewickelten Drähten (31a; 31c) geformt ist, und
der distale Endteil (46b; 46c) der Harzschicht (40d; 40e), in einer Querschnittansicht gesehen, in einen Raum (32; 32a) zwischen aneinandergrenzenden Drähten (31a; 31c) der mehreren Drähte (31a; 31c) des Wicklungskörpers (30a; 30b) eindringt.

6. Führungsdraht (1; 2; 3; 4; 5; 6; 7; 8) nach einem der Ansprüche 1 bis 5, wobei:
der Wicklungskörper (30; 30a; 30b; 30c) einen Kleindurchmesserabschnitt (62), der einen kleinen Wicklungsaußendurchmesser (D6) hat und sich an dem proximalen Endteil (42; 42a; 42b) der Harzschicht (40; 40a; 40b; 40c; 40d; 40e) befindet, einen Großdurchmesserabschnitt (66), der einen im Vergleich zum Kleindurchmesserabschnitt (62) größeren Wicklungsaußendurchmesser (D7) hat und sich an dem distalen Endteil (46; 46a; 46b; 46c) der Harzschicht (40; 40a; 40b; 40c; 40d; 40e) befindet, und einen Durchmesserzunahmeabschnitt (64) aufweist, der einen zur distalen Seite hin zunehmenden Wicklungsaußendurchmesser hat und sich an dem Verjüngungsteil (44; 44a; 44b) der Harzschicht (40; 40a; 40b; 40c; 40d; 40e) befindet, und
der Wicklungsaußendurchmesser (D7) des Großdurchmesserabschnitts (66) des Wicklungskörpers (30; 30a; 30b; 30c) größer ist als der große Außendurchmesser (D1) des proximalen Endteils (42; 42a; 42b) der Harzschicht (40; 40a; 40b; 40c; 40d; 40e) .

## Revendications

1. Fil-guide (1 ; 2 ; 3 ; 4 ; 5 ; 6 ; 7 ; 8), comprenant :
une tige centrale (10) ;
un corps de bobine (30 ; 30a ; 30b ; 30c) enroulé autour d'une périphérie externe de la tige centrale (10) ; et
une couche de résine (40 ; 40a ; 40b ; 40c ; 40d ; 40e) comprenant une partie d'extrémité proximale (42 ; 42a ; 42b) ayant un grand diamètre externe (D1), une partie d'extrémité distale (46 ; 46a ; 46b ; 46c) ayant un diamètre externe (D2 ; D3) plus petit que celui de la partie d'extrémité proximale (42 ; 42a ; 42b) dans un côté distal par rapport à la partie d'extrémité proximale (42 ; 42a ; 42b), la partie d'extrémité distale (46 ; 46a ; 46b ; 46c) étant située entre la tige centrale (10) et le corps de bobine (30 ; 30a ; 30b ; 30c), et une partie effilée (44 ; 44a ; 44b) ayant un diamètre externe diminuant vers le côté distal entre la partie d'extrémité proximale (42 ; 42a ; 42b) et la partie d'extrémité distale (46 ; 46a ; 46b ; 46c),
dans lequel le corps de bobine (30 ; 30a ; 30b ; 30c) est exposé sans être recouvert de la couche de résine (40 ; 40a ; 40b ; 40c ; 40d ; 40e) au niveau de la partie d'extrémité distale (46 ; 46a ; 46b ; 46c), **caractérisé en ce que**
la partie d'extrémité proximale (42 ; 42a ; 42b) couvre une périphérie externe du corps de bobine (30 ; 30a ; 30b ; 30c) sur toute la longueur de la partie d'extrémité proximale (42 ; 42a ; 42b).

2. Fil-guide (2 ; 3) selon la revendication 1, dans lequel la partie d'extrémité distale (46a) de la couche de résine (40a ; 40b) est fixée à la tige centrale (10).

3. Fil-guide (3 ; 4 ; 5 ; 6 ; 7 ; 8) selon la revendication 1 ou 2, dans lequel une portion renflée (50 ; 50a) ayant un diamètre externe (D4) supérieur au diamètre externe (D2 ; D3) de la partie d'extrémité distale (46 ; 46a ; 46b ; 46c) est prévue au niveau de la partie d'extrémité distale (46 ; 46a ; 46b ; 46c) de la couche de résine (40b ; 40c).

4. Fil-guide (2 ; 3 ; 4 ; 5 ; 6 ; 7 ; 8) selon l'une quelconque des revendications 1 à 3, comprenant en outre :
une portion de fixation (60) fixant la tige centrale (10), le corps de bobine (30 ; 30a ; 30b ; 30c), et la couche de résine (40 ; 40a ; 40b ; 40c ; 40d ; 40e) à une limite de la partie effilée (44 ; 44a ; 44b) de la couche de résine (40) et de la partie d'extrémité proximale (42 ; 42a ; 42b) de la couche de résine (40).

5. Fil-guide (6 ; 7) selon l'une quelconque des revendications 1 à 4, dans lequel :
le corps de bobine (30a ; 30b) est formé par un enroulement en spirale de fils multiples (31a ; 31c), et
la partie d'extrémité distale (46b ; 46c) de la couche de résine (40d ; 40e) pénètre dans un espace (32 ; 32a) entre des fils adjacents (31a ; 31c) des fils multiples (31a ; 31c) du corps de bobine (30a ; 30b) tel qu'observé dans une vue en coupe transversale.

6. Fil-guide (1 ; 2 ; 3 ; 4 ; 5 ; 6 ; 7 ; 8) selon l'une quelconque des revendications 1 à 5, dans lequel :
le corps de bobine (30 ; 30a ; 30b ; 30c) comprend une portion de petit diamètre (62) ayant un petit diamètre externe de bobine (D6) et étant située au niveau de la partie d'extrémité proximale (42 ; 42a ; 42b) de la couche de résine (40 ; 40a ; 40b ; 40c ; 40d ; 40e), une portion de grand diamètre (66) ayant un diamètre externe de bobine (D7) plus grand que celui de la portion de petit diamètre (62) et étant située au niveau de la partie d'extrémité distale (46 ; 46a ; 46b ; 46c) de la couche de résine (40 ; 40a ; 40b ; 40c ; 40d ; 40e), et une portion de diamètre croissant (64) ayant un diamètre externe de bobine augmentant vers le côté distal et étant située au niveau de la partie effilée (44 ; 44a ; 44b) de la couche de résine (40 ; 40a ; 40b ; 40c ; 40d ; 40e), et
le diamètre externe de bobine (D7) au niveau de la portion de grand diamètre (66) du corps de bobine (30 ; 30a ; 30b ; 30c) est plus grand que le grand diamètre externe (D1) au niveau de la partie d'extrémité proximale (42 ; 42a ; 42b) de la couche de résine (40 ; 40a ; 40b ; 40c ; 40d ; 40e).
